# EUROPEAN PATENT SPECIFICATION

(11) **EP 1 755 712 B1**
(45) Date of publication and mention of the grant of the patent: **10.01.2018**
(21) Application number: 05746482.8
(22) Date of filing: 27.05.2005
(51) Int. Cl.: A61M 5/20, A61M 5/32, A61M 5/24, A61M 5/315

(54) **INJECTION DEVICE**
INJEKTIONSVORRICHTUNG
DISPOSITIF D'INJECTION

(30) Priority: 28.05.2004 GB 0412050; 28.05.2004 GB 0412055; 28.05.2004 GB 0412053; 28.05.2004 GB 0412049; 28.05.2004 GB 0412061; 28.05.2004 GB 0412054; 28.05.2004 GB 0412057; 28.05.2004 GB 0412056; 28.05.2004 GB 0412048; 28.05.2004 GB 0412051; 06.04.2005 GB 0507010
(43) Date of publication of application: 28.02.2007
(73) Proprietor: Cilag GmbH International, 6300 Zug (CH)
(72) Inventor: HARRISON, Nigel David, Linton, Cambridge CB1 6YN (GB); HABESHAW, Rosemary Louise, Cambridge CB1 7TS (GB)
(74) Representative: Tunstall, Christopher Stephen
(86) International application number: PCT/GB2005/002135
(87) International publication number: WO 2005/115514

(56) References cited:
- EP-A- 0 516 473
- WO-A-01/87384
- GB-A- 2 388 033
- US-A- 4 521 237
- US-A- 5 779 668

## Description

### Background Technology

The present invention is concerned with injection devices of the type that include a housing, a syringe received within the housing, the syringe having a bore in which a discharge piston having a bore is inserted, a drive element and an actuator for advancing the drive element so as to advance the discharge piston and discharge the contents of the syringe through its discharge nozzle.

These days, nearly everything is manufactured by machines. In some circumstances, machines are more reliable that manual labour and are they are very much less expensive. However, there is one faculty of a production line worker that, hitherto, machines have been unable to reproduce, and are unlikely to be able to do so for a long time to come. If a production line worker drops a part of the thing being manufactured, he may pick it up or he may select another from the parts bin. If a part is damaged, he may discard it. If the assembly operation he is undertaking is complex, he will ensure that the parts are properly aligned and assembled. He does all of these things because he is able to adapt to different circumstances and to use his judgment. Machines cannot.

During automated assembling of injection devices of the type mentioned above, there are a number of critical steps that need to be performed accurately and properly. Difficulties in performing these steps may arise from tight component tolerances or from the use of fragile components, such as glass hypodermic syringes. The particular assembly step with which the present invention is concerned is that of getting the drive element into the bore of the syringe, to which a number of challenges attach. Firstly, to act on the outer diameter of the syringe piston, it is necessary for the drive element to have a flat end face that is a close fit in the syringe bore. Secondly, this flat end face presents the risk that the drive element may jam onto the end of the syringe, particularly if there is any misalignment between the components. Thirdly, if automation is used to assemble the devices a jam between the drive element and the syringe could result in damage to the device or even breakage of the syringe. Finally, even if this operation is done by hand, it is often necessary to do it blind.

WO01/87384A1 discloses an injection device with a flat-ended piston rod that is received in an ampoule for ejecting doses via a piston which connects with the flat end of the piston rod.

EP-A-0516473 discloses an auto-injector which would suffer from exactly these problems. A plunger is connected to a piston which travels within a capsule or syringe. The piston has a flat end face.

WO01/87384A1 discloses an injection device with a flat-ended piston rod that is received in an ampoule for ejecting doses via a piston which connects with the flat end of the piston rod.

US-A-4 521 237 discloses a glass dose syringe having a tubular glass barrel and a steel needle mounted thereto. The glass barrel has a flared opening formed by applying a barrel flaring operation to a cylindrical glass tube.

US-A-5779668 discloses a syringe system and a variety of embodiments of manually operated syringe devices. A plunger having a tapered end portion is provided within the syringe barrels.

US-A-5957897 describes a hollow needle applicator for cartridged drugs having provision for automatic needle retraction after the contents of the cartridge has been expelled.

### Summary of the Invention

It is an object of the present invention to provide a construction of injection device in which possible misalignment between the drive element and the syringe bore during assembly is already accounted for and does not so easily lead to a jam between the drive element and the syringe.

Accordingly, an injection device according to the present invention comprises:
a housing;
a syringe received within the housing, the syringe having a bore terminating at a forward end in a discharge nozzle and at a rearward end in a flared opening in which a discharge piston having a bore is inserted;
a drive element having a forward end consisting of a substantially flat edge region that is adapted to bear upon the discharge piston of the syringe and surrounds a projecting middle region that is adapted to be received in the bore of the discharge piston; and
an actuator for advancing the drive element so as to advance the discharge piston and discharge the contents of the syringe through the discharge nozzle,
wherein the projecting middle region tapers from the substantially flat edge region to a neb.

The combination of the flared opening to the syringe bore and the projecting middle region of the drive element allows the problems associated with mild misalignments of the two to be overcome. This is because the projecting middle region either passes straight into the end of the syringe, or it contacts the flared opening which guides it towards the centre of the syringe. Coupled with force sensors on the assembly machines, this arrangement will allow the appropriate automatic adjustment to be made to the alignment of the two parts.

Alternatively, and this is thought to be better because it does not rely on force sensors or other sophisticated modifications of the assembly machines, any misalignment is automatically corrected in full. This can be achieved if the flared opening in the syringe and the substantially flat edge and projecting middle regions of the forward end of the drive element are so shaped and dimensioned that axial misalignment between the syringe and the drive element during assembly of the injection device are corrected by, firstly, the projecting middle region of the forward end of the drive element riding up the flared opening of the syringe to a point at which, secondly, the substantially flat edge region of the forward end of the drive element makes contact with and rides up the flared opening of the syringe to align the drive element in the bore of the syringe.

The appropriate amount of flaring of the opening will depend upon a number of variables. Take a line formed by the intersection of the flared opening of the syringe and a plane that passes through the axis of the syringe bore. Preferably, this line possesses a radius of curvature of between 33% and 100% of the radius of the syringe bore. In the preferred embodiment, it possesses a radius of curvature of between 1 mm and 3 mm. This radius of curvature may be an instantaneous radius of curvature; it may be an average radius of curvature; it may be a minimum radius of curvature.

In embodiments in which the shape and dimensions of the flared opening in the syringe and the substantially flat edge and projecting middle regions of the forward end of the drive element are designed to correct axial misalignment in full, this radius of curvature should preferably be greater than the maximum radial extent of the substantially flat edge region of the forward end of the drive element. Better results are obtained if it is at least 50% greater than the maximum radial extent of the substantially flat edge region of the forward end of the drive element. Even better results follow if it is at least 70% greater.

To allow all directions of axial misalignment to be dealt with equally, the flared opening of the syringe is preferably substantially a surface of revolution about the axis of the syringe bore.

Normally, the forward end of the drive element has a cross-sectional area in the range 6.5 mm² to 110 mm², preferably 27.3 mm² ± 8%. Again, to allow all directions of axial misalignment to be dealt with equally, the forward end of the drive element may be substantially circular in cross-section. In that case, the forward end of the drive element normally has a radius in the range 1.45 mm to 5.9 mm, preferably 2.95 mm ± 4%. The substantially flat edge region of the forward end of the drive element may account for between 25% and 50% of the total area of the forward end of the drive element, preferably 37 ± 3 % of the total area of the forward end of the drive element.

Again, to allow all directions of axial misalignment to be dealt with equally, the substantially flat edge region of the forward end of the drive element may be substantially annular. Preferably, the inner diameter of the substantially flat annular region is 61 ± 2 % of the outer diameter.

Again, to allow all directions of axial misalignment to be dealt with equally, the projecting middle region of the forward end of the drive element is substantially circular in shape. For reasons that will be obvious, the projecting middle region of the forward end of the drive element tapers from the substantially flat edge region to a neb. It is thought that best results may be obtained if the projecting middle region of the forward end of the drive element tapers at an average angle of between 35 ± 10° to the longitudinal axis of the drive element. For example, the projecting middle region of the forward end of the drive element may be substantially conical or frustoconical with an included cone angle of 65 ± 5°.

### Brief Description of the Drawings

The present invention will now be described by way of example with reference to the accompanying drawings, in which:
figure 1 shows in perspective an injection device of the type to which the present invention is applicable;
figure 2 shows in section the injection device of figure 1 before actuation;
figure 3 shows an enlarged portion of figure 2; and
figure 4 is a perspective view of the drive element of figures 1-3.

### Detailed Description of the Preferred Embodiments

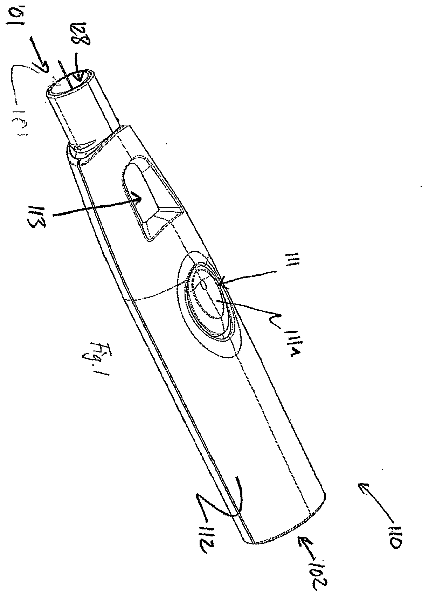

Figure 1 shows an injection device 110 having a housing 112 with a proximal end 101 and a distal end 102. All parts are injection-moulded. The housing 112 has a trigger 111 which projects through the housing 112 and which can be actuated by pressing down on its upper surface 111a. There is a indicator opening 113 in the housing located adjacent the proximal end 101.

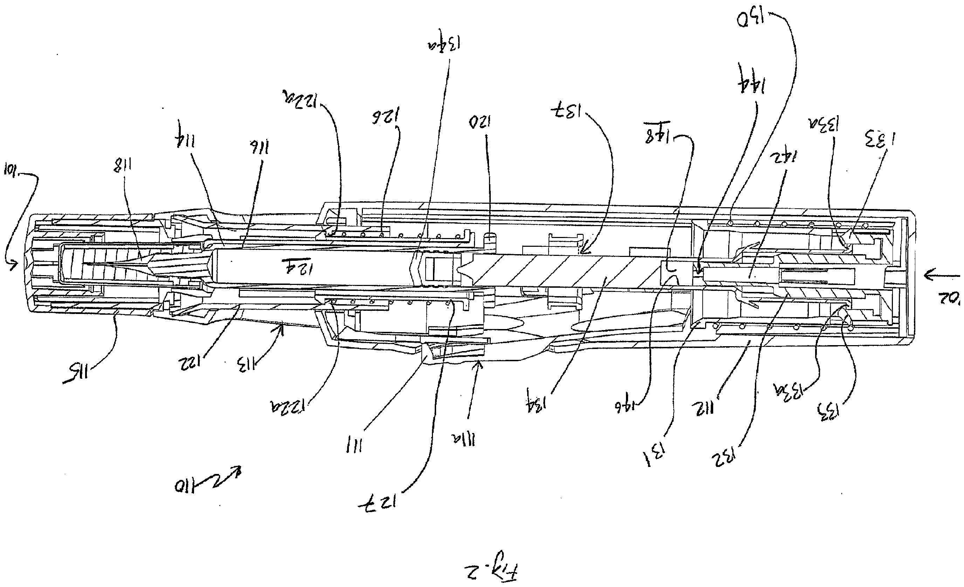

Figure 2 shows the housing 112 containing a hypodermic syringe 114 of conventional type, including a syringe body 116 terminating at one end in a hypodermic needle 118 and at the other in a flange 120. The conventional plunger and bung that would normally be used to discharge the contents of the syringe 114 manually have been removed and replaced with a drive element 134 which includes a bung 134a. This drive element 134 constrains a drug 124 to be administered within the syringe body 116. Whilst the syringe illustrated is of hypodermic type, this need not necessarily be so. Transcutaneous or ballistic dermal and subcutaneous syringes may also be used with the injection device of the present invention. As illustrated, the housing includes a return drive which here takes the form of a compression return spring 126 that biases the syringe 114 from an extended position in which the needle 118 extends from an aperture 128 in the housing 112 to a retracted position in which the discharge nozzle 118 is contained within the housing 112.

The housing 112 includes a support member which, as shown in figure 2, takes the form of a cylindrical insert 122. The cylindrical insert 122 has, on its inner surface, a support surface 122a which connects with one end of the return spring 126. The other end of the return spring 126 acts on the syringe 114 via a syringe carrier 127. The support surface 122a is provided, as shown in figure 2, by a rim on the inner surface of the cylindrical insert 122. The support surface 122a is positioned beyond the indicator opening 113 away from the proximal end 101 of the housing 112. The return spring 126 connects with the support surface 122a on its end which is located away from the proximal end 101 of the housing 112 and its other end acts on the syringe carrier 127 beyond the support surface 122a from the proximal end 101 of the housing 112. This way, the return spring 126, which surrounds the syringe 114 and syringe carrier 127, cannot be seen through the indicator opening 113 at any time before, during or after triggering of the injection device 110. The cylindrical insert 122 forms a window in the indicator opening 113 formed from transparent material so that the contents of the syringe 114 can be viewed through the indicator opening 113.

At the other end of the housing 112 is a forward drive, which here takes the form of a compression drive spring 130. Drive from the drive spring 130 is transmitted via a multi-component drive to the syringe 114 to advance it from its retracted position to its extended position and discharge its contents through the needle 118. The drive accomplishes this task by acting directly on the drug 124 and the syringe 114. Static friction between the drive element 134 and the syringe body 116 initially ensures that they advance together, until the return spring 126 bottoms out or the syringe body 116 meets some other obstruction (not shown) that retards its motion.

The multi-component drive between the drive spring 130 and the syringe 114 consists of three principal components. A drive sleeve 131 takes drive from the drive spring 130 and transmits it to a first drive element 132. This in turn transmits drive via a damping fluid to a second drive element, the drive element 134 already mentioned.

The first drive element 132 includes a hollow stem 140, the inner cavity of which forms a collection chamber 142 in communication with a vent 144 that extends from the collection chamber through the end of the stem 140. The second drive element 134 includes a blind bore 146 that is open at one end to receive the stem 140 and closed at the other. As can be seen, the bore 146 and the stem 140 defining a fluid reservoir 148, within which the damping fluid is contained.

The trigger 111, when operated, serves to decouple the drive sleeve 131 from the housing 112, allowing it to move relative to the housing 112 under the influence of the drive spring 130. The operation of the device is then as follows.

Initially, the drive spring 130 moves the drive sleeve 131, the drive sleeve 131 moves the first drive element 132 and the first drive element 132 moves the second drive element 134. The second drive element 134 moves and, by virtue of static friction and hydrostatic forces acting through the drug 124 to be administered, moves the syringe body 116 against the action of the return spring 126. The return spring 126 compresses and the hypodermic needle 118 emerges from the exit aperture 128 (not shown) of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug 124 to be administered are not sufficient to resist the full drive force developed by the drive spring 130, at this point the second drive element 134 begins to move within the syringe body 116 and the drug 124 begins to be discharged. Dynamic friction between the second drive element 134 and the syringe body 116 and hydrostatic and hydrodynamic forces now acting through the drug 124 to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, protrusions (not shown) on the first drive element 132 reach a constriction 137 within the housing 112. The constriction 137 moves the protrusions inwards so that the first drive element 136 is no longer coupled to the second drive element 134. Once this happens, the first drive element 136 no longer acts on the second drive element 134, allowing the first drive element 132 to move relative to the second drive element 134.

Because the damping fluid is contained within a reservoir 148 defined between the end of the first drive element 132 and the blind bore 146 in the second drive element 134, the volume of the reservoir 146 will tend to decrease as the first drive element 132 moves relative to the second drive element 134 when the former is acted upon by the drive spring 130. As the reservoir 148 collapses, damping fluid is forced through the vent 144 into the collection chamber 142. After release of the drive spring 130, some of the force exerted by the drive spring 130 does work on the damping fluid, causing it to flow though the constriction formed by the vent 144; the remainder acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir 148 of fluid is exhausted, flexible latch arms 133 linking the drive sleeve 131 with the first drive element 132 are no longer forced to engage the drive sleeve 131 by protrusions 133a on the second drive element 134. Once this happens, the drive sleeve 131 acts no longer on the first drive element 132, allowing them to move relative to each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 115 has been removed from the end of the housing 112. As can be seen from figure 2, the end of the syringe 114 is sealed with a boot 123.

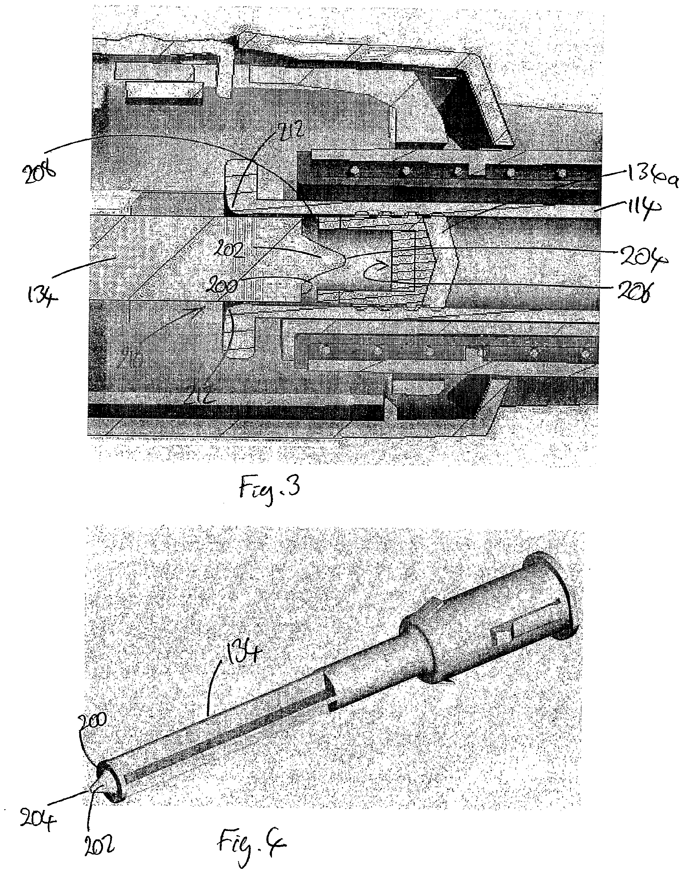

As discussed above and illustrated in figures 3 and 4, the drive element 134 terminates at its forward end in a flat annular region 200 that surrounds a substantially conical middle region 202 terminating in a neb 204. The rubber bung 134a possesses a central bore 206 in which the conical middle region 202 and the neb 204 are received and a skirt 208 that is borne upon by the annular flat region of the end of the drive element 134. The opening 210 in the rear of the glass syringe is flared, in this case by being provided with a radius at region 212. This radius is provided around the whole of the syringe bore opening and thus forms a surface of revolution.

The combination of the radius at regions 212 of the opening 210 to the syringe bore and the projecting conical middle region 202 and the neb 204 of the drive element allows misalignments of the two to be managed. This is because the conical middle region 202 and the neb 204 either pass straight into the opening 210 of the syringe, or contact the radius at regions 212, which guides them towards the centre of the syringe bore. The radius at regions 212 and the substantially flat annular region and the central conical portion 202 and neb 204 of the drive element 134 are so shaped and dimensioned that axial misalignment between the syringe 114 and the drive element 134 during assembly of the injection device are corrected by, firstly, the conical middle region 202 of the drive element riding up the radius at region 212 to a point at which, secondly, the substantially flat annular region 200 of the drive element 134 also makes contact with and rides up the radius at region 212, to align the drive element 134 in the bore of the syringe 114.

In this preferred embodiment of the invention, the inner diameter of the bore of the syringe 114 is 6.35 ± 0.1 mm. The appropriate radius to be provided at regions 212 depends a number of variables. A typical radius may be between 33% and 100% of the radius of the syringe bore; in this preferred embodiment, it possesses a radius of curvature of between 1 mm and 3 mm. Since in this embodiment, the shape and dimensions of the flared opening in the syringe and the substantially flat annular and conical middle regions of the drive element 134 are designed to correct axial misalignment in full, this radius of curvature should preferably be greater than the maximum radial extent of the substantially flat annular region of the drive element 134. Better results are obtained if it is at least 50% greater than the maximum radial extent of the substantially flat edge region of the forward end of the drive element. Even better results follow if it is at least 70% greater. In this embodiment, a radius of 2 mm is preferred, which compares with a maximum radial extent of the substantially flat annular region 200 of the drive element 134 of about 1.15 mm.

The drive element 134 itself has a diameter at its forward of 5.9 ± 0.22 mm (i.e. ± 4%) and thus its cross-sectional area is 27.3 mm² ± 8%. The diameter of the base of the conical middle portion 202 is 3.6 mm ± 4%, which accounts for about 63% of the total area of the end of the drive element 134; the remaining 37% is accounted for by the flat annular region 200. The conical middle region of the forward end of the drive element has an included cone angle of 65 ± 5°, which means that the sides of the cone taper at about 32.5° from the longitudinal axis of the drive element 134. These various preferred dimensions and angles make up a device that has been found to work extremely well.

A drive element 134 shaped in the way described has further advantages. If it is desired to reduce the dose volume of the device, this is easily done by inserting, during the assembly operation, an additional drive element between element 134 and the bung 134a. At its forward end, the additional drive element should reproduce exactly the shape and dimensions of the drive element 134; at its rearward end, it should reproduce the shape and dimensions of the bore and skirt of the bung 134a. Thus, to the drive element 134, the additional drive element will be indistinguishable from the bung 134a; to the bung 134a, it will be indistinguishable from the drive element 134.

## Claims

1. An injection device (110) comprising:
a housing (112);
a syringe (114) received within the housing (112), the syringe (114) having a bore terminating at a forward end in a discharge nozzle (118) and at a rearward end in an opening (210) in which a discharge piston (134a) having a bore (206) is inserted;
a drive element (134) having a forward end consisting of a substantially flat edge region (200) that is adapted to bear upon the discharge piston (134a) of the syringe (114); and
an actuator for advancing the drive element (134) so as to advance the discharge piston (134a) and discharge the contents of the syringe (114) through the discharge nozzle (118),
**characterised in that** the opening (210) is flared, that the flat edge region (200) surrounds a projecting middle region (202) that is adapted to be received in the bore (206) of the discharge piston, and that the projecting middle region (202) tapers from the substantially flat edge (200) region to a neb (204).

2. An injection device (110) according to claim 1 in which the flared opening in the syringe and the substantially flat edge and projecting middle regions of the forward end of the drive element are so shaped and dimensioned that axial misalignment between the syringe and the drive element during assembly of the injection device are corrected by, firstly, the projecting middle region of the forward end of the drive element riding up the flared opening of the syringe to a point at which, secondly, the substantially flat edge region of the forward end of the drive element rides up the flared opening of the syringe to align the drive element in the bore of the syringe.

3. An injection device (110) according to claim 2 in which the line formed by the intersection of the flared opening of the syringe and a plane that passes through the axis of the syringe bore possesses a radius of curvature of between 33% and 100% of the radius of the syringe bore.

4. An injection device (110) according to claim 2 in which the line formed by the intersection of the flared opening of the syringe and a plane that passes through the axis of the syringe bore possesses a radius of curvature of between 1 mm and 3 mm.

5. An injection device (110) according to claim 3 or claim 4 in which the said radius of curvature is an average radius of curvature.

6. An injection device (110) according to claim 3 or claim 4 in which the said radius of curvature is a minimum radius of curvature.

7. An injection device (110) according to any one of claims 3-6 in which the said radius of curvature is greater than the maximum radial extent of the substantially flat edge region of the forward end of the drive element.

8. An injection device (110) according to claim 7 in which the said radius of curvature is at least 50% greater than the maximum radial extent of the substantially flat edge region of the forward end of the drive element.

9. An injection device (110) according to claim 7 in which the said radius of curvature is at least 70% greater than the maximum radial extent of the substantially flat edge region of the forward end of the drive element.

10. An injection device (110) according to any preceding claim in which the flared opening of the syringe is substantially a surface of revolution about the axis of the syringe bore.

11. An injection device (110) according to any preceding claim in which the forward end of the drive element has a cross-sectional area in the range 6.5 mm² to 110 mm².

12. An injection device (110) according to claim 11 in which the forward end of the drive element has a cross-sectional area of 27.3 mm² ± 8%.

13. An injection device (110) according to any preceding claim in which the forward end of the drive element is substantially circular in cross-section.

14. An injection device (110) according to claim 13 in which the forward end of the drive element has a radius in the range 1.45 mm to 5.9 mm.

15. An injection device (110) according to claim 14 in which the forward end of the drive element has a radius of 2.95 mm ± 4%.

16. An injection device (110) according to any preceding claim in which the substantially flat edge region of the forward end of the drive element accounts for between 25% and 50% of the total area of the forward end of the drive element.

17. An injection device (110) according to claim 16 in which the substantially flat edge region of the forward end of the drive element accounts for 37 ± 3 % of the total area of the forward end of the drive element.

18. An injection device (110) according to any preceding claim in which the substantially flat edge region of the forward end of the drive element is substantially annular.

19. An injection device (110) according to claim 18 in which the inner diameter of the substantially flat annular region is 61 ± 2 % of the outer diameter.

20. An injection device (110) according to any preceding claim in which the projecting middle region of the forward end of the drive element is substantially circular in shape.

21. An injection device (110) according to any one of the preceding claims in which the projecting middle region of the forward end of the drive element tapers at an average angle of between 35 ± 10° to the longitudinal axis of the drive element.

22. An injection device (110) according to claim 20 or claim 21 in which the projecting middle region of the forward end of the drive element is substantially conical or frustoconical.

23. An injection device (110) according to claim 22 in which the conical or frustoconical region of the forward end of the drive element has an included cone angle of 65 ± 5°.

## Patentansprüche

1. Injektionsvorrichtung (110), umfassend:
ein Gehäuse (112);
eine in dem Gehäuse (112) aufgenommene Spritze (114), wobei die Spritze (114) eine Bohrung aufweist, die an dem vorderen Ende in einer Ausstoßdüse (118) und an dem hinteren Ende in einer Öffnung (210) endet, in die ein Ausstoßkolben (134a), der eine Bohrung (206) aufweist, eingeführt ist;
ein Treibelement (134) mit einem vorderen Ende, das aus einem im wesentlichen flachen Randbereich (200) besteht, der dafür ausgelegt ist, an dem Ausstoßkolben (134a) der Spritze (114) anzuliegen; und
einen Aktor zum Vorschieben des Treibelements (134), um den Ausstoßkolben (134a) vorzuschieben und den Inhalt der Spritze (114) durch die Ausstoßdüse (118) auszustoßen,
**dadurch gekennzeichnet, dass** die Öffnung (210) aufgeweitet ist, dass der flache Randbereich (200) einen vorstehenden Mittelbereich (202) umgibt, der dafür ausgelegt ist, in der Bohrung (206) des Ausstoßkolbens aufgenommen zu werden, und dass der vorstehende Mittelbereich (202) von dem im Wesentlichen flachen Randbereich (200) zu einer Spitze (204) zuläuft.

2. Injektionsvorrichtung (110) gemäß Anspruch 1, wobei die aufgeweitete Öffnung in der Spritze und der im Wesentlichen flache Rand- und vorstehende Mittelbereich des vorderen Endes des Treibelements so geformt und bemessen sind, dass axiale Fehlausrichtung zwischen der Spritze und dem Treibelement während des Zusammensetzens der Injektionsvorrichtung dadurch korrigiert wird, dass zuerst der vorstehende Mittelteil des vorderen Endes des Treibelements in der aufgeweiteten Öffnung der Spritze bis zu einem Punkt vorwandert, bei dem als zweites der im Wesentlichen flache Randbereich des vorderen Endes des Treibelements in der aufgeweiteten Öffnung der Spritze vorwandert, um das Treibelement in der Bohrung der Spritze auszurichten.

3. Injektionsvorrichtung (110) gemäß Anspruch 2, wobei die Linie, die von dem Schnitt der aufgeweiteten Öffnung der Spritze mit einer Ebene gebildet wird, die durch die Achse der Spritzenbohrung verläuft, einen Krümmungsradius von zwischen 33 % und 100 % des Radius der Spritzenbohrung aufweist.

4. Injektionsvorrichtung (110) gemäß Anspruch 2, wobei die Linie, die von dem Schnitt der aufgeweiteten Öffnung der Spritze mit einer Ebene gebildet wird, die durch die Achse der Spritzenbohrung verläuft, einen Krümmungsradius von zwischen 1 mm und 3 mm aufweist.

5. Injektionsvorrichtung (110) gemäß Anspruch 3 oder Anspruch 4, wobei der Krümmungsradius ein mittlerer Krümmungsradius ist.

6. Injektionsvorrichtung (110) gemäß Anspruch 3 oder Anspruch 4, wobei der Krümmungsradius ein minimaler Krümmungsradius ist.

7. Injektionsvorrichtung (110) gemäß einem der Ansprüche 3-6, wobei der Krümmungsradius größer als die größte radiale Abmessung des im Wesentlichen flachen Randbereichs des vorderen Endes des Treibelements ist.

8. Injektionsvorrichtung (110) gemäß Anspruch 7, wobei der Krümmungsradius wenigstens 50 % größer als die größte radiale Abmessung des im Wesentlichen flachen Randbereichs des vorderen Endes des Treibelements ist.

9. Injektionsvorrichtung (110) gemäß Anspruch 7, wobei der Krümmungsradius wenigstens 70 % größer als die größte radiale Abmessung des im Wesentlichen flachen Randbereichs des vorderen Endes des Treibelements ist.

10. Injektionsvorrichtung (110) gemäß einem der vorstehenden Ansprüche, wobei die aufgeweitete Öffnung der Spritze im Wesentlichen eine Rotationsfläche um die Achse der Spritzenbohrung ist.

11. Injektionsvorrichtung (110) gemäß einem der vorstehenden Ansprüche, wobei das vordere Ende des Treibelements eine Querschnittsfläche in dem Bereich von 6,5 mm² bis 110 mm² aufweist.

12. Injektionsvorrichtung (110) gemäß Anspruch 11, wobei das vordere Ende des Treibelements eine Querschnittsfläche von 27,3 mm² ± 8 % aufweist.

13. Injektionsvorrichtung (110) gemäß einem der vorstehenden Ansprüche, wobei das vordere Ende des Treibelements einen im Wesentlichen kreisförmigen Querschnitt aufweist.

14. Injektionsvorrichtung (110) gemäß Anspruch 13, wobei das vordere Ende des Treibelements einen Radius in dem Bereich von 1,45 mm bis 5,9 mm aufweist.

15. Injektionsvorrichtung (110) gemäß Anspruch 14, wobei das vordere Ende des Treibelements einen Radius von 2,95 mm ± 4 % aufweist.

16. Injektionsvorrichtung (110) gemäß einem der vorstehenden Ansprüche, wobei der im Wesentlichen flache Randbereich des vorderen Endes des Treibelements zwischen 25 % und 50 % der Gesamtfläche des vorderen Endes des Treibelements bildet.

17. Injektionsvorrichtung (110) gemäß Anspruch 16, wobei der im Wesentlichen flache Randbereich des vorderen Endes des Treibelements 37 ± 3 % der Gesamtfläche des vorderen Endes des Treibelements bildet.

18. Injektionsvorrichtung (110) gemäß einem der vorstehenden Ansprüche, wobei der im Wesentlichen flache Randbereich des vorderen Endes des Treibelements im Wesentlichen ringförmig ist.

19. Injektionsvorrichtung (110) gemäß Anspruch 18, wobei der Innendurchmesser des im Wesentlichen flachen ringförmigen Bereichs 61 ± 2 % des Außendurchmessers beträgt.

20. Injektionsvorrichtung (110) gemäß einem der vorstehenden Ansprüche, wobei der vorstehende Mittelbereich des vorderen Endes des Treibelements im Wesentlichen kreisförmig ist.

21. Injektionsvorrichtung (110) gemäß einem der vorstehenden Ansprüche, wobei der vorstehende Mittelbereich des vorderen Endes des Treibelements mit einem mittleren Winkel von 35 ± 10° zu der Längsachse des Treibelements zuläuft.

22. Injektionsvorrichtung (110) gemäß Anspruch 20 oder Anspruch 21, wobei der vorstehende Mittelbereich des vorderen Endes des Treibelements im Wesentlichen kegelförmig oder kegelstumpfförmig ist.

23. Injektionsvorrichtung (110) gemäß Anspruch 22, wobei der kegelförmige oder kegelstumpfförmige Bereich des vorderen Endes des Treibelements einen eingeschlossenen Kegelwinkel von 65 ± 5° aufweist.

## Revendications

1. Dispositif d'injection (110) comprenant :
un logement (112) ;
une seringue (114) accueillie à l'intérieur du logement (112), la seringue (114) ayant un alésage se terminant au niveau d'une extrémité avant dans une buse d'évacuation (118) et au niveau d'une extrémité arrière dans une ouverture (210) dans laquelle un piston d'évacuation (134a) possédant un alésage (206) est introduit ;
un élément d'entraînement (134) possédant une extrémité avant consistant en une région de bord sensiblement plat (200) qui est conçue pour supporter le piston d'évacuation (134a) de la seringue (114) ; et
un actionneur permettant de faire avancer l'élément d'entraînement (134) de façon à faire avancer le piston d'évacuation (134a) et à évacuer le contenu de la seringue (114) par la buse d'évacuation (118),
**caractérisé en ce que** l'ouverture (210) est évasée, que la région de bord plat (200) entoure une région intermédiaire saillante (202) qui est conçue pour être accueillie dans l'alésage (206) du piston d'évacuation, et que la région intermédiaire saillante (202) décroît depuis la région de bord sensiblement plat (200) vers un bec (204).

2. Dispositif d'injection (110) selon la revendication 1 dans lequel l'ouverture évasée dans la seringue et la région de bord sensiblement plat et la région intermédiaire saillante de l'extrémité avant de l'élément d'entraînement sont façonnées et dimensionnées de sorte qu'un mauvais alignement axial entre la seringue et l'élément d'entraînement pendant le montage du dispositif d'injection soit corrigé, tout d'abord, par la région intermédiaire saillante de l'extrémité avant de l'élément d'entraînement qui remonte l'ouverture évasée de la seringue jusqu'à un point auquel, ensuite, la région de bord sensiblement plat de l'extrémité avant de l'élément d'entraînement remonte l'ouverture évasée de la seringue afin d'aligner l'élément d'entraînement dans l'alésage de la seringue.

3. Dispositif d'injection (110) selon la revendication 2 dans lequel la ligne formée par l'intersection de l'ouverture évasée de la seringue et un plan qui passe par l'axe de l'alésage de la seringue possède un rayon de courbure compris entre 33 % et 100 % du rayon de l'alésage de la seringue.

4. Dispositif d'injection (110) selon la revendication 2 dans lequel la ligne formée par l'intersection de l'ouverture évasée de la seringue et un plan qui passe par l'axe de l'alésage de la seringue possède un rayon de courbure compris entre 1 mm et 3 mm.

5. Dispositif d'injection (110) selon la revendication 3 ou la revendication 4 dans lequel ledit rayon de courbure est un rayon de courbure moyen.

6. Dispositif d'injection (110) selon la revendication 3 ou la revendication 4 dans lequel ledit rayon de courbure est un rayon de courbure minimum.

7. Dispositif d'injection (110) selon l'une quelconque des revendications 3 à 6 dans lequel ledit rayon de courbure est supérieur à l'étendue radiale maximum de la région de bord sensiblement plat de l'extrémité avant de l'élément d'entraînement.

8. Dispositif d'injection (110) selon la revendication 7 dans lequel ledit rayon de courbure est au moins 50 % supérieur à l'étendue radiale maximum de la région de bord sensiblement plat de l'extrémité avant de l'élément d'entraînement.

9. Dispositif d'injection (110) selon la revendication 7 dans lequel ledit rayon de courbure est au moins 70 % supérieur à l'étendue radiale maximum de la région de bord sensiblement plat de l'extrémité avant de l'élément d'entraînement.

10. Dispositif d'injection (110) selon l'une quelconque des revendications précédentes dans lequel l'ouverture évasée de la seringue est sensiblement une surface de révolution sur l'axe de l'alésage de la seringue.

11. Dispositif d'injection (110) selon l'une quelconque des revendications précédentes dans lequel l'extrémité avant de l'élément d'entraînement possède une coupe transversale dans la plage de 6,5 mm² à 110 mm².

12. Dispositif d'injection (110) selon la revendication 11 dans lequel l'extrémité avant de l'élément d'entraînement possède une surface de coupe transversale de 27,3 mm² ± 8 %.

13. Dispositif d'injection (110) selon l'une quelconque des revendications précédentes dans lequel l'extrémité avant de l'élément d'entraînement possède une coupe transversale sensiblement circulaire.

14. Dispositif d'injection (110) selon la revendication 13 dans lequel l'extrémité avant de l'élément d'entraînement possède un rayon dans la plage de 1,45 mm à 5,9 mm.

15. Dispositif d'injection (110) selon la revendication 14 dans lequel l'extrémité avant de l'élément d'entraînement possède un rayon de 2,95 mm ± 4 %.

16. Dispositif d'injection (110) selon l'une quelconque des revendications précédentes dans lequel la région de bord sensiblement plat de l'extrémité avant de l'élément d'entraînement représente entre 25 % et 50 % de la surface totale de l'extrémité avant de l'élément d'entraînement.

17. Dispositif d'injection (110) selon la revendication 16 dans lequel la région de bord sensiblement plat de l'extrémité avant de l'élément d'entraînement représente 37 ± 3 % de la surface totale de l'extrémité avant de l'élément d'entraînement.

18. Dispositif d'injection (110) selon l'une quelconque des revendications précédentes dans lequel la région de bord sensiblement plat de l'extrémité avant de l'élément d'entraînement est sensiblement annulaire.

19. Dispositif d'injection (110) selon la revendication 18 dans lequel le diamètre intérieur de la région annulaire sensiblement plate est 61 ± 2 % du diamètre extérieur.

20. Dispositif d'injection (110) selon l'une quelconque des revendications précédentes dans lequel la région intermédiaire saillante de l'extrémité avant de l'élément d'entraînement est de forme sensiblement circulaire.

21. Dispositif d'injection (110) selon l'une quelconque des revendications précédentes dans lequel la région intermédiaire saillante de l'extrémité avant de l'élément d'entraînement décroît selon un angle moyen de 35 ± 10° par rapport à l'axe longitudinal de l'élément d'entraînement.

22. Dispositif d'injection (110) selon la revendication 20 ou la revendication 21 dans lequel la région intermédiaire saillante de l'extrémité avant de l'élément d'entraînement est sensiblement conique ou tronconique.

23. Dispositif d'injection (110) selon la revendication 22 dans lequel la région conique ou tronconique de l'extrémité avant de l'élément d'entraînement possède un angle de cône inclus de 65 ± 5°.
